(19) Europäisches Patentamt / European Patent Office / Office européen des brevets

(11) **EP 2 124 821 B1**

(12) **EUROPÄISCHE PATENTSCHRIFT**

(45) Veröffentlichungstag und Bekanntmachung des Hinweises auf die Patenterteilung:
**16.11.2011   Patentblatt 2011/46**

(21) Anmeldenummer: **07786490.8**

(22) Anmeldetag: **01.08.2007**

(51) Int Cl.:
**A61F 2/16** $^{(2006.01)}$

(86) Internationale Anmeldenummer:
**PCT/EP2007/006802**

(87) Internationale Veröffentlichungsnummer:
**WO 2008/101522 (28.08.2008 Gazette 2008/35)**

(54) **AKKOMODATIVE INTRAOKULARLINSE**

ACCOMMODATIVE INTRAOCULAR LENS

LENTILLE INTRAOCULAIRE ACCOMMODATRICE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **25.02.2007   DE 102007009502**

(43) Veröffentlichungstag der Anmeldung:
**02.12.2009   Patentblatt 2009/49**

(73) Patentinhaber:
• **Detmers, Ulfert**
  **26524 Hage (DE)**
• **Meyer, Matthias, Dr. med.**
  **26845 Nortmoor (DE)**

(72) Erfinder:
• **Detmers, Ulfert**
  **26524 Hage (DE)**
• **Meyer, Matthias, Dr. med.**
  **26845 Nortmoor (DE)**

(74) Vertreter: **Kaminski Harmann**
  **Patentanwälte Est.**
  **Austrasse 79**
  **9490 Vaduz (LI)**

(56) Entgegenhaltungen:
**EP-A- 1 475 055     EP-A1- 0 356 050
FR-A- 2 666 735     US-A1- 2004 082 995**

EP 2 124 821 B1

**Beschreibung**

Die Erfindung betrifft eine akommodative Intraokularlinse für phake, d. h. naturentsprechend linsenhaltige Augen nach dem Oberbegriff des Anspruchs 1.

Zur Korrektur von Refraktionsfehlern des menschlichen Auges sind im Stand der Technik Kunstlinsen bekannt, die zu der natürlichen Augenlinse durch Implantation hinzugefügt werden. In den gängigen Verfahren erfolgt die Einpflanzung einer solchen intraokularen Linse als zusätzliche Kunstlinse zu der natürlichen, biologischen Augenlinse. Die Kunstlinse soll dabei einen Refraktionsfehler ausgleichen und eine Brille ersetzen, bzw. verträglicher machen, wobei die Einpflanzung üblicherweise über einen Hornhautschnitt mit faltbaren oder starren Kunstlinsen erfolgt.

In den Verfahren des Stands der Technik werden üblicherweise zwei Implantationsorte gewählt:

　　　　a.) Vor der Iris als so genannte Vorderkammerlinse
　　　　b.) Zwischen Iris und natürlicher Linse als sogenannte Hinterkammerlinse

In FR 2666735 wird eine Vorderkammerlinse offenbart, die aus zwei Linsenteilen mit zwischenliegendem Hohlraum besteht.

Bisherige Linsen, die zur Implantation in Augen mit natürlicher Linse benutzt werden, bestehen aus Silikon, Hydrogel oder aus starren wie flexiblen Acrylaten. Diese Materialien sind vielfach bewährt. Bislang konnte damit zwar ein Refraktionsfehler ausgeglichen werden, jedoch findet die im Alter zunehmende Akkommodationsschwäche (Alterssichtigkeit/Presbyopie) in den phaken Linsen keine Korrektur, so dass wiederum eine zusätzliche Korrektur, z. B. durch eine Brille erforderlich wird.

Eine Aufgabe der Erfindung besteht darin, eine verbesserte intraokulare Kunstlinse bereitzustellen.

Eine weitere Aufgabe besteht darin, eine intraokulare Kunstlinse zur Verfügung zu stellen, die, in das Auge implantiert, sowohl den Mangel der Akkommodation durch Alter in erheblichem Maße reduziert als auch optische Refraktionsfehler ausgleicht.

Diese Aufgaben werden durch die Gegenstände des Anspruchs 1 oder der abhängigen Ansprüche gelöst bzw. die Lösungen weitergebildet.

Die Erfindung basiert darauf, eine Intraokularlinse akkommodativ, insbesondere pneumatisch, auszubilden, so dass eine zusätzlich zur natürlichen Augenlinse hinzufügbare Kunstlinse entsteht, die in das Auge chirurgisch implantiert, sowohl den Mangel der Akkommodation durch Alter in erheblichem Maße reduziert als auch optische Refraktionsfehler ausgleicht und dadurch möglichst lange eine hohe Sehschärfe ohne anwendbare Hilfsmittel wie Brillen ermöglicht.

Erfindungsgemäß wird dabei die Akkommodationsfähigkeit des Auges, die auch im Alter noch vorhanden ist, genutzt, wobei durch die erfindungsgemässen optischen und mechanischen Eigenschaften der Kunstlinse die Effektivität der im Alter reduzierten Akkommodation um ein Vielfaches erhöht wird. Der Effekt einer verbesserten Wirkung der im Alter vorhandenen Restakkommodation wird dadurch erzielt, dass eine flexible Kunstlinse mit einem Brechungsindex nahe 1, die umgeben ist von optisch wirksamen Medien mit grösserem Brechungsindices, z.B. von ca. 1,3 bis 1,4, durch die akkommodative Bewegung bzw. Auslenkung der natürlichen Linse in eine konkave Form gebracht wird. Die Gesamtbrechkraft des Auges wird also dadurch erhöht, indem ein künstliches Medium mit sehr geringem Brechungsindex durch die akkommodative Bewegung der natürlichen Linse angetrieben formverändert wird, in eine zunehmend konkave Form getrieben wird und somit die Gesamtbrechkraft des Auges erhöht, womit eine effektive Nahfokussierung ermöglicht wird.

Grundsätzlich bestimmt die Differenz der Brechungsindizes der einzelnen optischen Elemente die Größe der Gesamtbrechkraft eines optischen Systems. Für die natürliche Linse im Kontakt mit dem umgebenden Kammerwasser liegt die Differenz der Brechungsindizes bei ca. 0,07. Die Differenz der Brechungsindizes zwischen einer flexiblen flüssigen bzw. gasförmigen Linse liegt materialabhängig bei bis zu 0,386. Entsprechend wird eine akkommodative Linsenwölbung die bei kleiner Brechungsindexdifferenz von 0,053 nur eine Brechkraftänderung von 0,5 Dioptrien bewirkt, bei einer Indexdifferenz von 0,386 um ein vielfaches effizienter.

Die erfindungsgemässe phake Intraokularlinse wird nachfolgend rein beispielhaft anhand einer Hinterkammerlinse näher beschrieben oder erläutert.

Eine solche Intraokularlinse als Hinterkammerlinse kann zwischen der natürlichen biologischen Linse und die Regenbogenhaut implantiert werden. Die linsenzugewandte Seite der Kunstlinse besteht dann aus einer dünnen Schicht des Kunstlinsenträgermaterials. Eingeschlossen in die Kunstlinse ist ein Hohlraum, welcher auch mit Gas, Flüssigkeit, Öl oder auch einem Feststoff gefüllt werden kann, für die Funktionsweise dieser erfindungsgemäßen Kunstlinse idealerweise mit einem Gas. Die der Regenbogenhaut zugewandte Seite besteht aus einer dickeren Schicht des Kunstlinsenträgermaterials oder auch aus einem anderen Material. Im peripheren Bereich der Kunstlinse sind Möglichkeiten des Volumenausgleichs der Hohlraumfüllung geschaffen, um die Binnenlinse wirken lassen zu können. Bei gasförmiger Ausgestaltung des Binnenraums der Kunstlinse werden atmosphärische Druckschwankungen hierdurch kompensiert.

Durch diese Ausgestaltung bleibt bei akkommodationsbedingter Radienverkleinerung der Innenseite der Kunstlinse der Radius der Außenseite weitgehend stabil und die Binnenlinse verformt sich.

Damit setzt sich die erfindungsgemässe Kunstlinse aus drei funktional unterschiedlichen Komponenten oder Abschnitten zusammen, die vorzugsweise aus biologisch inerten Materialien ausgebildet sind, die chemisch nicht miteinander reagieren und für die Substanzen der Kunstlinse völlig diffusionsdicht, sowie optisch klar sind:

- Eine flexible Komponente, die durch einen Flüssigkeitsfilm getrennt, der natürlichen Linse anliegt, so dass akkommodative Bewegungen der natürlichen Linse von dieser flexiblen Komponente direkt und nach Möglichkeit in vollem Umfang nachvollzogen werden. Eine hydrophile Oberflächengestaltung der flexiblen Komponente kann zur Gewährleistung einer stabilen Flüssigkeitsschicht zwischen natürlicher Linse und Kunstlinse erfolgen. Die Form der flexiblen Komponente wird dabei vorteilhafterweise so gestaltet, dass die Dicke vom Zentrum zur Peripherie hin kontinuierlich zunimmt.

- Eine Zwischenkomponente mit einem Brechungsindex, der unter dem der umgebenden optischen Medien (natürliche Linse, Kammerwasser) liegt. Diese Komponente liegt der flexiblen Komponente direkt an und folgt den Bewegungen der flexiblen Komponente in voller Auslenkung. Für den Zweck der Brechkraftverstärkung bei geringer Restakkommodation sind Medien mit geringem, insbesondere sehr geringem Brechungsindex erforderlich. Diese Bedingungen werden bevorzugt durch Gase und einige Flüssigkeiten erfüllt. Erfindungsgemäss kommen bevorzugt Gase bzw. fluide Medien zum Einsatz, die nicht durch die verwendeten Linsenmaterialien diffusionsfähig sind. Die Forderung nach biologisch inerten Gasen bzw. Flüssigkeiten kann in diesem Bereich dahingehend eingeschränkt werden, dass auch solche Substanzen eingesetzt werden können die zwar nicht inert aber völlig unbedenklich sind. Grundsätzlich sind alle niedrig siedenden Substanzen geeignet, die bei den physiologischen Temperaturen und Drücken innerhalb des menschlichen Auges in den gasförmigen Zustand übergehen und die biologisch, mindestens jedoch für das verwendete Linsenmaterial chemisch inert sind, wobei Diffusionsdichtigkeit des Linsenmaterials gewährleistet sein muss. Hierzu gehören beispielsweise

  - Kohlendioxid,
  - Edelgase, d.h. Argon, Neon, Krypton, Xenon, sowie
  - Schwefelhexafluorid,
  - Octafluorpentan,
  - Perfluorbutan,
  - Perfluorpentan,
  - Octafluorcyclopentan,
  - Perfluorcyclopentan,
  - Perfluotmethylcyclopentan,
  - Perfluorcyclophexan,
  - Hydrofluorether,
  - Perfluorketon oder
  - Perfluorcyclohexan.

  Einige dieser Substanzen weisen zwar eine Toxizität auf, sind aber bei ausreichender Dichtigkeit der umschliessenden Materialien, zumindest theoretisch, einsetzbar.

- Eine starre oder vergleichsweise unflexible Komponente. Diese Komponente schließt die gasförmige, bzw. flüssige Komponente durch eine völlig dichte zirkuläre Verbindung mit der flexiblen Komponente in der Peripherie ein. Die periphere Verdickung der flexiblen Komponente wird bei der unflexiblen Komponente durch eine zentrale Verdickung kompensiert. Zusammen mir der flexiblen Komponente bildet diese die Form der gasförmigen bzw. flüssigen Komponente. Die unflexible Komponente ist weiterhin dafür geeignet Korrekturen für Fehlsichtigkeiten aufzunehmen. Durch das Zusammenfügen von fester und flexibler Komponente entsteht ein spaltförmiger Raum zwischen beiden, der die optisch aktive Zone zur Akkommodationsverstärkung durch Formveränderung der gasförmigen bzw. flüssigen Komponente enthält.

Im Zustand der Ferneinstellung des Auges ist die gasförmige bzw. flüssige Komponente, die im folgenden als Gaslinse bezeichnet wird, optisch unwirksam, d.h. die Grenzflächen von flexibler und unflexibler Komponente stehen zweckmäßigerweise parallel zueinander.

Durch eine akkommodative Bewegung der natürlichen Linse überträgt sich deren verringerter Krümmungsradius auf den Krümmungsradius der flexiblen Komponente und somit erhält die Gaslinse in der Folge eine konkave Charakteristik. Die Formveränderung der Gaslinse würde dabei unter Umständen ohne Kompensationsmechanismen zu einer Drucksteigerung innerhalb der Gaslinse führen, wodurch die Übertragung der Änderungen der Krümmungsradien der natürlichen Linse auf die Gaslinse behindert würden, da Druckdifferenzen zu überwinden wären.

Zur Druckkompensation sind zwei grundsätzliche unterschiedliche Mechanismen möglich. Zum Einen kann die überschüssige Gas- oder Flüssigkeitsmenge aus dem Spaltraum in die Peripherie durch periphere Öffnungen in einen entsprechenden ebenfalls randständigen Raum abgeführt werden, wo diese dann bedarfsentsprechend zur Rückführung bereitsteht, oder durch eine flexible Verbindung zwischen flexibler Komponente und unflexibler Komponente wird eine Auslenkung der unflexiblen Komponente in Richtung der Iris ermöglicht, wodurch eine Druckentlastung gewährleistet ist. Aufgrund der sehr geringen Volumina sind diese Ausgleichsbewegungen hinsichtlich der räumlichen Verhältnisse, die innerhalb des Auges bestehen, unproblematisch. Da lageabhängig ein gewisser hydrostatischer Druckgradient bestehen kann, wird die unflexible Komponente bei Akkommodation nicht an allen Punkten gleich weit in Richtung Iris verlagert, wodurch ein leichter prismatischer Effekt entstehen kann. Da dieser Effekt beidseitig und gleichsinnig auftritt, ist eine Wahrnehmung beim binokularen Sehvorgang nicht möglich.

Bei der Ableitung der verdrängten Gas- oder Flüssigkeitsmengen in periphere Membranräume der Kunstlinse ist zwar eine konstante Positionierung der unflexiblen Komponente zur flexiblen Komponente möglich, jedoch sind die Fertigungsschritte zur Herstellung einer solchen Linse erheblich aufwendiger. Da der Druckausgleich durch periphere Öffnungen erfolgt, lässt sich ggf. der Ausgleich extern durch beispielsweise Einsatz von Laserstrahlung manipulieren und somit ggf. das Ausmaß variieren

Um die unflexible Komponente beweglich zu applizieren kann die Peripherie der flexiblen Komponente doppellagig ausgeführt werden, was beispielsweise durch die größere periphere Dicke gewährleistbar ist. Zum Zentrum hin verschmelzen beide Lagen. Die unflexible Komponente wird an der iriszugewandten Lage befestigt. Bei Druckkompensation werden die beiden Lagen voneinander abgehoben, bzw. wieder einander angenähert. Die Doppellagigkeit der Peripherie der flexiblen Komponente lässt sich bei der Herstellung an einem drehbankähnlichen Gerät durch einen Schnitt, dessen Kanten abzurunden sind, vergleichsweise einfach erstellen.

Im Fall der Ausführung der Kunstlinse mit Gas ist insbesondere das Diffusionsverhalten zu berücksichtigen. Hier können entsprechend diffusionsdichte Materialien (z.B. hydrophobe Acrylate) verwendet werden sowie ergänzend oder alternativ die bereits erwähnten nicht oder nur extrem langsam diffundierenden Gase, wie z.B. grossatomige Edelgase oder molekulare Verbindungen. Gase die unter physiologischen Bedingungen entstehen und in die Linse hinein diffundieren können, müssen schon bei der Herstellung der Linse in solchen Konzentrationen eingebracht werden, dass sie am Implantationsort im Diffusionsgleichgewicht mit den dort vorkommenden Gasen stehen.

Da die Randzonen der Kunstlinse reflektierende Strukturen aufweisen, können reflektionsunterdrückende oder -mindernde Maßnahmen, wie z.B. die Schwärzung des Linsenmaterials zu vorgenommen werden. Zur Vermeidung störender Lichtreflexe kann im Bereich der Ausgleichsräume im peripheren Bereich das Material lichtdicht behandelt werden.

Die erfindungsgemässe Kunstlinse wird nachfolgend anhand von in der Zeichnung schematisch dargestellten Ausführungsbeispielen rein beispielhaft näher beschrieben. Im einzelnen zeigen

Fig. 1a-b    die schematische Darstellung der Akkommodation einer natürlichen Linse;

Fig.2a-b    die schematische Darstellung der Akkommodation einer ersten Ausführungsform der erfindungsgemässen Linse;

Fig.3    die schematische Darstellung einer zweiten Ausführungsform der erfindungsgemässen Linse und

Fig.4    den Einfluß einer variablen Gaslinse auf die Brechkraft der biologischen Linse im Augeninneren.

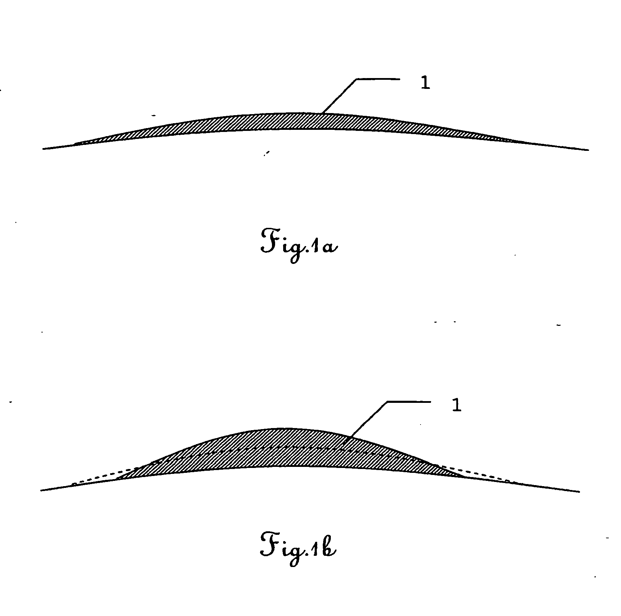

Fig. 1a-b zeigt die schematische Darstellung der Akkommodation einer natürlichen Linse 1, wobei Fig. 1a den entspannten, nicht akkommodierten Ruhezustand der Linse 1 und Fig.1b den akkommodierten Zustand darstellen.

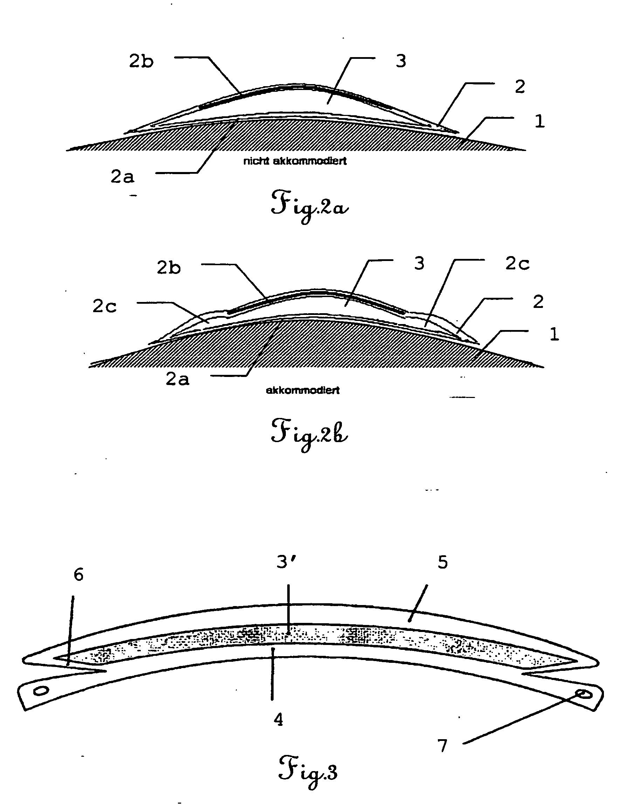

In den Fig.2a-b erfolgt die schematische Darstellung der Akkommodation einer ersten Ausführungsform einer erfindungsgemäßen akkommodativen Kunst- oder Intraokularlinse mit einem einstückigen Linsenkörper 2 und einem darin eingearbeiteten Hohlraum 3 zur Verstärkung der refraktiven Wirkung des akkommodativen Restvermögens der natürlichen Linse 1. Der Linsenkörper 2 weist ein erstes flächiges Linsenteil 2a und ein zweites flächiges Linsenteil 2b auf, wobei das erste Linsenteil 2a zur direkten oder indirekten Kontaktierung der natürlichen Linse des menschlichen Auges ausgebildet ist und dem nach außen orientierten zweite Linsenteil 2b gegenüberliegt. Fig.2a zeigt den entspannten, nicht akkomodierten Ruhezustand der Kunstlinse und Fig.2b den akkomodierten Zustand. Die Vorder- und Rückfläche des Hohlraums 3 können dabei auch so ausgebildet werden, dass sie im nichtakkomodierten Zustand, d.h. ohne extern erzeugte Deformation, parallel zueinander stehen, wobei das erste Linsenteil 2a im zentralen Bereich die geringste Stärke und das zweite Linsenteil 2b im zentralen Bereich seine größte Stärke aufweist.

Hierfür ist es notwendig, dass die Kunstlinse weitgehend der vorderseitigen Krümmung der natürlichen Linse 1 angepasst wird, um sich dieser möglichst gleichsinnig anzuschmiegen und flächig zu kontaktieren. Kommt es zu der in Fig.1b dargestellten Krümmungsveränderung der Linsenvorderfläche der natürlichen Linse 1, so erfährt die Kunstlinse mit ihrer Rückfläche, d.h. dem ersten Linsenteil 2a, dieselben Veränderungen, wobei die Kunstlinse auf einem dünnen Flüssigkeitsfilm auf der natürlichen Augenlinse schwimmt. Durch entsprechende. Ausgestaltung, z.B. mittels einer Beschichtung mit einem Material mit erhöhtem Brechungsindex, erfährt die Kunstlinsenvorderfläche, d.h. d.h. das zweite Linsenteil 2b, eine stärkere Brechkraft und die Brechkrafterhöhung der natürlichen Linse 1 wird verstärkt. Dies hat zur Folge, dass die restliche erhaltene Akkommodationsfähigkeit so gesteigert wird, dass eine mittlerweile verlorene oder gefährdete Lesefähigkeit wiedererlangt werden kann. Das erste Linsenteil 2a kann auch eine hydrophile Beschichtung zur Stabilisierung einer Flüssigkeitsschicht zwischen Kunstlinse und natürlicher Linse 1 aufweisen.

Das Trägermaterial des Linsenkörpers 2 kann hierbei aus üblichen durchsichtigen Kunststoffen bestehen, wie sie bisher in der operativen Augenheilkunde gebräuchlich sind (Silikon, Acrylate, Hydrogel etc.) Durch Ausbildung von unterschiedlichen Materialstärken für erstes Linsenteil 2a und zweites Linsenteil 2b kann erreicht werden, daß sich der dickere zweite Linsenteil 2b durch die Restakkommodation des Auges in geringerem Masse deformiert als der vergleichsweise dünner gehaltene erste Linsenteil 2a. Dabei können zur Erzielung einer hohen mechanischen Stabilität die peripheren Bereiche von erstem und/oder zweitem Linsenteil 2a, 2b dicker ausgebildet sein als die entsprechenden zentralen Bereiche, insbesondere mit einem kontinuierlichen und stufenlosen Übergang.

Der zusätzliche akkommodative Effekt resultiert zudem aus einer Hohlraumgestaltung der Kunstlinse, wobei der Hohlraum 3 vorzugsweise mit transparenter Flüssigkeit, transparentem Gas oder auch einem transparenten und deformierbaren Feststoff mit inerten Eigenschaften und optischer Reinheit gefüllt ist. Der Effekt der verbesserten Wirkung der im Alter vorhandenen Restakkommodation wird dadurch erzielt, dass der - bspw. gasgefüllte - Hohlraum 3 als in die Trägerlinse eingeschlossene weitere Linse gelten kann und durch die akkommodative Bewegung bzw. Auslenkung in eine konkavere Form gebracht wird. Der Hohlraum 3 wird dabei so ausgebildet ist, dass bei einer Radiusveränderung des ersten Linsenteils 2a durch akkommodative Bewegungen der natürlichen Linse 1 im Auge eine demgegenüber vergleichsweise geringere Radiusveränderung des zweiten Linsenteils 2b erfolgt, so daß eine Konkavitätsveränderung des Hohlraums 3 resultiert.

Um hierdurch eine Zunahme der Gesamtbrechkraft des Auges zu erreichen, wird das optische Material der inneren Flüssigkeits- oder Gaslinse so zu so gewählt, dass dessen Brechungsindex kleiner ist als der der natürlichen Linse und des umgebenden Kammerwassers. Diese Forderung wird von Gasen und einigen Flüssigkeiten erfüllt.

Bei entsprechender Ausgestaltung der Intraokularlinse bilden sich im akkommodierten Zustand am Rand angeordneten Taschen oder periphere Kompensationsvolumina 2c zum Volumenausgleich der Hohlraumfüllung bei der Verformung der Gesamtstruktur. Hierdurch kann der Innendruck konstant gehalten werden, so daß eine Druckkompensation bewirkt wird. Das Linsenmaterial kann so gewählt werden, daß ein Füllgas im Hohlraum 3 als Gaslinse nicht durch das Linsenmaterial diffundiert. Ein weiterer bei der Auswahl des Linsenmaterials zu berücksichtigender Punkt ist die Durchlässigkeit für andere Gase, die in gelöster oder an Blutbestandteilen angelagerter Form im menschlichen Körper vorkommen, wie insbesondere Sauerstoff oder Stickstoff. Hier ist es vorteilhaft, ein für diese Gase durchlässiges Linsenmaterial zur Druck-/Volumenkompensationen durch Ein-und Ausdiffusion zu wählen.

Fig.3 zeigt eine zweite Ausführungsform der erfindungsgemässen Linse mit zusammengesetztem Aufbau in schematischer Darstellung. In diesem Fall ist die Struktur nicht einstückig ausgeführt sondern weist eine flexible Membran 4 als die natürliche Linse des Auges kontaktierende Komponente und damit als erster Linsenteil auf. Ihr gegenüber ist eine vergleichsweise unflexible Frontplatte 5 als zweiter Linsenteil angeordnet, wobei die Komponenten durch eine flexible Verbindung 6 fixiert werden. Diese starre Frontplatte 5 erlaubt beispielsweise auch die Einarbeitung von optischen Korrekturen zur Kompensation von Brechungsfehlern des Auges.

Im Inneren der Linse ist wiederum eine Hohlraum 3' ausgebildet, der eine Gas- oder Luftfüllung aufweisen kann. Zur Erhöhung der mechanischen Stabilität kann die Kunstlinse eine Verstärkungsstruktur 7 aufweisen, z.B. in Form eines hohlen, umlaufenden segmentierten Rings. In der Peripherie können in ähnlicher Weise auch Ballastgewichte, insbesondere ringförmige Gewichte in den Randzonen, zur Kompensation von gasfüllungsbedingten Auftriebseffekten untergebracht werden. Werden diese Ballastgewichte durch eine mechanisch stabilisierende flexible Verbindung untereinander verkettet, so erfolgt eine Stabilisierung des gesamten Linsenkörpers.

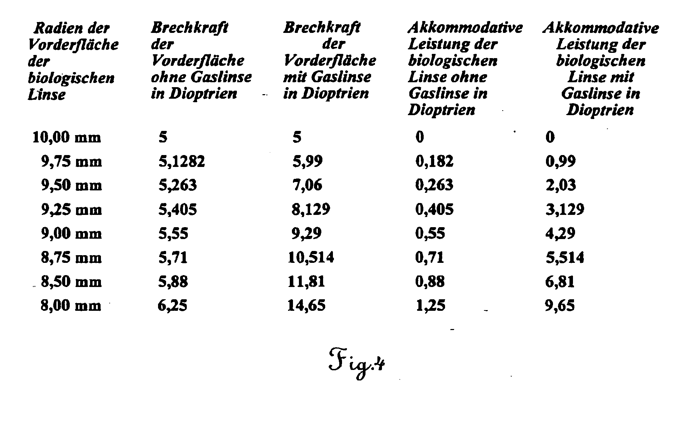

Der Einfluß einer variablen Gaslinse auf die Brechkraft der biologischen Linse im Augeninneren wird in Fig.4 anhand der Ergebnisse einer beispielhaften Berechnung verdeutlicht.

Die zugrundeliegende Kalkulation geht davon aus, dass eine flexible gasförmige Linse zwischen Iris und biologischer Linse positioniert wird. Diese gasförmige Linse wird in einer flexiblen Membran eingeschlossen und in ihrer Brechkraft variiert. Die Veränderung der Brechkraft der Gaslinse wird dadurch erreicht, dass die Verstärkung der Krümmung der biologischen Linse bei der Akkommodation genutzt wird, die Gaslinse in eine konkave Form zu bringen. Die der Iris zugewandten Seite der Gaslinse bleibt hierbei konstant. Für die Linsenberechnungen wurde ein normalsichtiges Auge angenommen, welches in der Ferne keinerlei optische Korrektur benötigt. Entsprechend liegen die Vorder- und Rückfläche der Gaslinse parallel zueinander, sodass keine optische Wirkung im Sinne einer Sammel- oder Zerstreuungswirkung entsteht (Fernakkommodation). Ebenso sind die optischen Parameter des flexiblen und des starren Teils der Kunstlinse so dimensioniert, dass kein Sammel- oder Zerstreuungseffekt in der Gesamtheit entsteht, d.h. die Funktion der Kunstlinse beruht nur auf der Formveränderung der Gaslinse.

Da also der flexible und der starre Teil der Kunstlinse keinen Sammel- oder Zerstreuungseffekt haben, wurden diese Anteile in der Linsenberechnung nicht berücksichtigt. Zwar hat die Dicke der Kunstlinse einen gewissen Anteil auf die Gesamtbrechung, jedoch ist diese Wirkung um ein vielfaches kleiner als die Gesamtwirkung der Kunstlinse bei Formveränderung der Gaslinse, sodass das Prinzip der Kunstlinse hierdurch nicht entscheidend tangiert wird. Lediglich zur Berechnung der Wirkung der Dicke der Kunstlinse ist die Kenntnis der dazugehörigen Brechungsindizes erforderlich. Dies bedeutet letztlich, daß ein vereinfachtes Modell ohne Berücksichtigung der Kunstlinsendicke verwendet wird.

Zu den Materialien ist grundsätzlich festzustellen, daß in erster Linie in der Augenchirurgie übliche flexible und ggf. unflexible Substanzen eingesetzt werden können, wie z.B. Acrylate oder Siliconverbindungen. Für den unflexiblen Teil der Kunstlinse gilt, dass dieser lediglich funktionell unflexibel sein muss, d. h. es kann durchaus ein flexibles Material verwendet werden, solange es die Bedingung der Formstabilität bei akkomodierender Formänderung der Gaslinse erfüllt.

Die zu erwartende Verstärkung der Brechkraftänderung bei Akkommodation wird genutzt, um die Restakkommodation im Alter bei Presbyopie zu unterstützen. Gegenübergestellt wird die Wirkung der Restakkommodation ohne und mit Gaslinse in Dioptrien, wobei hierbei zunächst die Brechkraft an der vorderen Linsenfläche ohne Gaslinse, d.h. unter physiologischen Bedingungen, ausgehend vom Zustand der Fernakkommodation (r = 10 mm) berechnet wird. Der Krümmungsradius der Linsenvorderfläche wird hierbei in Abstufungen von 0,5 bzw. 0,25 mm verringert. Es werden jeweils 8 Berechnungen ohne und mit Gaslinse durchgeführt.

Der Berechnung liegen hierbei folgende Beziehungen zugrunde

$$f = \frac{n_l \cdot r_l}{n_l - n_{kw}} \tag{1}$$

$$D = \frac{n_l}{f} \tag{2}$$

was nach Einsetzen

$$D = \frac{n_l \cdot (n_l - n_{kw})}{n_l \cdot r_l} \tag{3}$$

ergibt. Dieser Ausdruck beschreibt die brechende Wirkung der biologischen Linse im Kammerwasser an ihrer Vorderfläche. Soll die brechende Wirkung der biologischen Linse an ihrer Vorderfläche gemeinsam mit der vorgesetzten Gaslinse ermittelt werden, ergeben sich hieraus folgende Beziehungen:

$$D = \frac{n_{gl} \cdot (n_{gl} - n_{kw})}{n_{gl} \cdot r_{gl}} + \frac{n_l \cdot (n_l - n_{gl})}{n_l \cdot r_l} \tag{4}$$

$$D = \frac{(n_{gl} - n_{kw})}{r_{gl}} + \frac{(n_l - n_{gl})}{r_l} \tag{5}$$

Hierbei werden folgende Bezeichnungen verwendet

f        = Brennweite (hier der Anteil der Linsenvorderfläche an der Gesamtbrennweite)

D = Dioptrien
$n_{kw}$ = Brechungsindex des Kammerwassers(1,336)
$n_l$ = Brechungsindex der biologischen Linse (1,386)
$n_{gl}$ = Brechungsindex der Gaslinse(1,0003)
$r_{gl}$ = Radius der Gaslinse an der Vorderfläche zur Iris hin(10 mm)
$r_l$ = Radius der Gaslinse an der Hinterfläche, bzw. der Radius der biologischen Linse an der Vorderfläche(maximal 10 mm)

Die resultierenden Ergebnisse sind in Fig.4 in tabellarischer Form dargestellt und verdeutlichen, daß eine erfindungsgemäße gas- oder flüssigkeitsgefüllte Linse eine effektive Akkommodationshilfe darstellt.

## Patentansprüche

1. Phake Intraokularlinse, insbesondere zur Presbyopiekorrektur, mit einem Linsenkörper (2) aus optisch transparentem Linsenmaterial, vorzugsweise aus Silikon oder Acrylat, mit einem ersten flächigen Linsenteil (2a,4) und einem gegenüberliegenden zweiten flächigen Linsenteil (2b, 5), und zwischen dem ersten Linsenteil (2a,4) und dem zweiten Linsenteil (2b,5) ein Hohlraum (3,3') im Linsenkörper (2) ausgebildet ist **dadurch gekennzeichnet, dass** der erste Linsenteil (2a,4) zur Kontaktierung der natürlichen Linse des menschlichen Auges ausgebildet ist, wobei der erste Linsenteil (2a,4) eine gegenüber dem zweiten Linsenteil (2b,5) höhere Deformierbarkeit aufweist .

2. Intraokularlinse nach Anspruch 1,
**dadurch gekennzeichnet, dass**
der Hohlraum (3,3') so ausgebildet ist, dass bei einer Krümmungsradiusveränderung des ersten Linsenteils (2a,4) durch akkommodative Bewegungen der natürlichen Linse (1) im Auge eine demgegenüber vergleichsweise geringere oder keine Krümmungsradiusveränderung des zweiten Linsenteils(2b,5) erfolgt, so daß eine Veränderung der Konkavität des Hohlraums (3,3') resultiert.

3. Intraokularlinse nach Anspruch 1 oder 2,
**dadurch gekennzeichnet, dass**
der Hohlraum (3,3') mit

- einer Flüssigkeit,
- einem Gas oder
- einem deformierbaren Feststoff als optisch transparentem Medium gefüllt ist, wobei das Medium einen gegenüber dem ersten und/oder zweiten Linsenteil (2a,2b,4,5) geringeren Brechungsindex aufweist.

4. Intraokularlinse nach einem der vorangehenden Ansprüche,
**dadurch gekennzeichnet, dass**
zur Kompensation von Brechungsfehlern des Auges Korrekturen in den zweiten Linsenteil (2b,5) eingearbeitet sind.

5. Intraokularlinse nach einem der vorangehenden Ansprüche,
**dadurch gekennzeichnet, daß**
Vorder- und Rückfläche des Hohlraums (3,3') im nichtakkomodierten Zustand parallel zueinander stehen, wobei der erste Linsenteil (2a,4) im zentralen Bereich die geringste Stärke und der zweite Linsenteil (2b,5) im zentralen Bereich seine größte Stärke aufweist.

6. Intraokularlinse nach einem der vorangehenden Ansprüche,
**dadurch gekennzeichnet, daß**
der Linsenkörper (2) einstückig und mit einer ersten Linsenfläche als erstem Linsenteil(2a) und einer zweiten Linsenfläche als zweitem Linsenteil (2b) ausgebildet ist.

7. Intraokularlinse nach einem der vorangehenden Ansprüche 1 bis 5,
**dadurch gekennzeichnet, daß**
die Linse einen zusammengesetzten Aufbau mit einer flexiblen Membran als erstem Linsenteil (4) und einer funktionell unflexible Frontplatte als zweitem Linsenteil (5) aufweist.

8. Intraokularlinse nach einem der vorangehenden Ansprüche,

**dadurch gekenazeichnet, daß**
die Linse Kompensationsvolumina (2c) zur Druckkompensation aufweist, insbesondere in der Peripherie des Hohlraums (3,3').

9. Intraokularlinse nach einem der vorangehenden Ansprüche,
**dadurch gekennzeichnet, daß**
periphere Bereiche von erstem und/oder zweitem Linsenteil (2a,2b,4,5) dicker ausgebildet sind als die entsprechenden zentralen Bereiche, insbesondere mit einem kontinuierlichen und stufenlosen übergang.

10. Intraokularlinse nach einem der vorangehenden Ansprüche,
**dadurch gekennzeichnet, daß**
der erste Linsenteil (4) in der Peripherie zirkulär doppellagig als hohler, umlaufender Ring ausgebildet ist.

11. Intraokularlinse nach einem der vorangehenden Ansprüche,
**dadurch gekennzeiehnet, daß**
die peripheren Bereiche des Linsenkörpers (2) reflexmindernd und/oder lichtundurchlässig ausgebildet sind, insbesondere durch Schwärzung.

12. Intraokularlinse nach einem der vorangehenden Ansprüche,
**dadurch gekennzeichnet, daB**
der erste Linsenteil (2a,4) eine hydrophile Beschichtung zur Stabilisierung einer Flüssigkeitsschicht zwischen Kunstlinse und natürlicher Linse aufweist.

13. Intraokularlinse nach einem der vorangehenden Ansprüche,
**dadurch gekennzeichnet, daß**
der Hohlraum (3,3') als Gaslinse mit einem nicht durch das binsenmaterial diffundierenden Füllgas ausgebildet ist.

14. Intraokularlinse nach Anspruch 13,
**dadurch gekennzeichnet, daß**
das Füllgas wenigstens eines der folgenden Gase ist

- ■ Kchlendioxid,
- ■ Ec.elgase, d.h. Argon, Neon, Krypton, Xenon, sowie
- ■ Schwefelhexafluorid,
- ■ Octafluorpenran,
- ■ Perfluorbutan,
- ■ Perfluorpentan,
- ■ Octafluorcyclopentan,
- ■ Perfluorcyclopentan,
- ■ Perfluormethylcyclopentan,
- ■ Perfluorcyclohexan,
- ■ Hydrofluorether,
- ■ Perfluorketon oder
- ■ Perfluorcyclohexan.

15. Intraokularlinse nach einem der vorangehenden Ansprüche,
**dadurch gekennzeichnet, daß**
das Linsenmaterial zur Druck-/Volumenkompensationen durch Ein- und Ausdiffusion für atmosphärische Gase, die in gelöster oder an Blutbestandteilen angelagerter Form im menschlichen Körper vorkommen, wie insbesondere Sauerstoff oder Stickstoff, durchlässig ist.

16. Intraokularlinse nach einem der vorangehenden Ansprüche,
**gekennzeichnet durch**
Ballastgewichte, insbesondere ringförmige Gewichte, in den Randzonen zur Kompensation von gasfüllungsbedingten Auftriebseffekten.

17. Intraokularlinse nach Anspruch 16,
**dadurch gekennzeichnet, daß**

die Ballastgewichte durch eine mechanisch stabilisierende Fixierung untereinander verbunden sind.

**Claims**

1.  Phakic intraocular lens, in particular for presbyopia correction, having a lens body (2) composed of optically transparent lens material, preferably of silicone or acrylate, and having a first flat lens portion (2a, 4) and an opposite second flat lens portion (2b, 5), and a cavity (3, 3') being formed in the lens body (2) between the first lens portion (2a, 4) and the second lens portion (2b, 5), **characterized in that** the first lens portion (2a, 4) is designed to contact the natural lens of the human eye, the first lens part (2a, 4) having a higher deformability than the second lens part (2b, 5).

2.  Intraocular lens according to Claim 1, **characterized in that** the cavity (3, 3') is designed such that, a change in the radiative curvature of the first lens portion (2a, 4), caused by accommodative movements of the natural lens (1) in the eye, results in no change in the radiative curvature of the second lens portion (2b, 5) or in a change in the radiative curvature of the second lens portion (2b, 5) that is less in comparison therewith, such that a change in the concavity of the cavity (3, 3') results.

3.  Intraocular lens according to Claim 1 or 2, **characterized in that** the cavity (3, 3') is filled with

    - a liquid,
    - a gas, or
    - a deformable solid
    as optically transparent medium, the medium having a smaller refractive index in comparison with the first and/or second lens portion (2a, 2b, 4, 5).

4.  Intraocular lens according to one of the preceding claims, **characterized in that** corrections are incorporated in the second lens portion (2b, 5) in order to compensate refractive errors of the eye.

5.  Intraocular lens according to one of the preceding claims, **characterized in that** front and rear surfaces of the cavity (3, 3') are parallel to one another in the non-accommodated state, the first lens portion (2a, 4) exhibiting the minimum thickness in the central area, and the second lens portion (2b, 5) exhibiting its maximum thickness in the central area.

6.  Intraocular lens according to one of the preceding claims, **characterized in that** the lens body (2) is designed in one piece and with a first lens surface as first lens portion (2a) and a second lens surface as second lens portion (2b).

7.  Intraocular lens according to one of the preceding claims 1 to 5, **characterized in that** the lens exhibits a composite design having a flexible membrane as first lens portion (4) and a functionally inflexible front plate as second lens portion (5).

8.  Intraocular lens according to one of the preceding claims, **characterized in that** the lens has compensation volumes (2c) for pressure compensation, in particular in the periphery of the cavity (3, 3').

9.  Intraocular lens according to one of the preceding claims, **characterized in that** peripheral areas of the first and/or second lens portion (2a, 2b, 4, 5) is of thicker design than the corresponding central areas, in particular with a continuous and stepless transition.

10. Intraocular lens according to one of the preceding claims, **characterized in that** the first lens portion (4) is designed in the periphery as a circular double layer in the form of a hollow, circumferential ring.

11. Intraocular lens according to one of the preceding claims, **characterized in that** the peripheral areas of the lens body (2) are designed to reduce reflection and/or to be opaque, in particular by blackening.

12. Intraocular lens according to one of the preceding claims, **characterized in that** the first lens portion (2a, 4) has a hydrophilic coating for stabilizing a liquid layer between the artificial lens and natural lens.

13. Intraocular lens according to one of the preceding claims, **characterized in that** the cavity (3, 3') is designed as a gas lens having a fill gas which does not diffuse through the lens material.

**14.** Intraocular lens according to Claim 13, **characterized in that** the fill gas is at least one of the following gases

- carbon dioxide,
- inert gases, for example argon, neon, krypton, xenon, and
- sulphur hexafluoride,
- octafluoropentane,
- perfluorobutane,
- perfluoropentane,
- octafluorocyclopentane,
- perfluorocyclopentane,
- perfluoromethylcyclopentane,
- perfluorocyclohexane,
- hydrofluoroether,
- perfluoroketone, or
- perfluorocyclohexane.

**15.** Intraocular lens according to one of the preceding claims, **characterized in that** for the purpose of pressure/volume compensations by inward and outward diffusion, the lens material is permeable to atmospheric gases which occur in the human body in dissolved form or in a form attached to the constituents of the blood such as, in particular, oxygen or nitrogen.

**16.** Intraocular lens according to one of the preceding claims, **characterized by** ballast weights, in particular annular weights, in the edge zones for the purpose of compensating buoyancy effects caused by gas filling.

**17.** Intraocular lens according to Claim 16, **characterized in that** the ballast weights are interconnected by being fixed in a mechanically stabilizing fashion.

**Revendications**

**1.** Lentille intraoculaire phakique, en particulier pour la correction de la presbytie, avec un corps de lentille (2) réalisé en matériau de lentille optiquement transparent, de préférence en silicone ou en acrylate, avec une première partie de lentille (2a, 4) plate et une deuxième partie de lentille (2b, 5) plate opposée et un espace creux (3, 3') est réalisé dans le corps de lentille (2), entre la première partie de lentille (2a, 4) et la deuxième partie de lentille (2b, 5), **caractérisée en ce que**
la première partie de lentille (2a, 4) est réalisée pour l'établissement du contact de la lentille naturelle de l'oeil humain, où
la première partie de lentille (2a, 4) présente une déformabilité plus élevée que celle de la deuxième partie de lentille (2b, 5).

**2.** Lentille intraoculaire selon la revendication 1, **caractérisée en ce que**
l'espace creux (3, 3') est réalisé de manière que, dans le cas d'une modification du rayon de courbure de la première partie de lentille (2a, 4), une modification du rayon de courbure relativement faible, voire aucune modification du rayon de courbure, de la deuxième partie de lentille (2b, 5) ne s'effectue grâce à des déplacements accommodatifs de la lentille naturelle (1) dans l'oeil, de manière qu'en résulte une modification de la concavité de l'espace creux (3, 3').

**3.** Lentille intraoculaire selon la revendication 1 ou 2,
**caractérisée en ce que**
l'espace creux (3, 3') est rempli, en tant que milieu optiquement transparent :

- d'un liquide,
- d'un gaz, ou
- d'une substance solide déformable,

le milieu présentant un indice de réfraction plus faible que celui de la première et/ou deuxième partie de lentille (2a, 2b, 4, 5).

**4.** Lentille intraoculaire selon l'une des revendications précédentes,

**caractérisée en ce que**

des corrections sont apportées par usinage dans la deuxième partie de lentille (2b, 5), afin d'apporter une compensation des défauts de réfraction de l'oeil.

5.  Lentille intraoculaire selon l'une des revendications précédentes,
    **caractérisée en ce que**,
    à l'état non accommodé, la face avant et la face arrière de l'espace creux (3, 3') sont parallèles entre elles, la première partie de lentille (2a, 4) présentant l'épaisseur minimale dans la zone centrale, et la deuxième partie de lentille (2b, 5) présentant son épaisseur maximale dans la zone centrale.

6.  Lentille intraoculaire selon l'une des revendications précédentes,
    **caractérisée en ce que**
    le corps de lentille (2) est réalisé d'une seule pièce et avec une première face de lentille en tant que première partie de lentille (2a, 4) et une deuxième face de lentille en tant que deuxième partie de lentille (2b, 5).

7.  Lentille intraoculaire selon l'une des revendications 1 à 5 précédentes,
    **caractérisée en ce que**
    la lentille présente une structure composite, avec une membrane flexible en tant que première partie de lentille (4) et une plaque avant, fonctionnellement inflexible, en tant que deuxième partie de lentille (5).

8.  Lentille intraoculaire selon l'une des revendications précédentes,
    **caractérisée en ce que**
    la lentille présente des volumes de compensation (2c) pour la compensation de pression, en particulier dans la périphérie de l'espace creux (3, 3').

9.  Lentille intraoculaire selon l'une des revendications précédentes,
    **caractérisée en ce que**
    des zones périphériques de la première et/ou de la deuxième partie de lentille (2a, 2b, 4, 5) sont de réalisation plus épaisse que les zones centrales correspondantes, en particulier avec une transition continue et sans échelonnement.

10. Lentille intraoculaire selon l'une des revendications précédentes,
    **caractérisée en ce que**
    la première partie de lentille (4a) est, dans la périphérie, de réalisation circulaire à double couche, sous forme d'anneau de pourtour creux.

11. Lentille intraoculaire selon l'une des revendications précédentes,
    **caractérisée en ce que**
    les zones périphériques du corps de lentille (2) sont réalisées pour diminuer la réflexion et/ou être opaques à la lumière, en particulier grâce à un noircissement.

12. Lentille intraoculaire selon l'une des revendications précédentes,
    **caractérisée en ce que**
    la première partie de lentille (2a, 4) présente un revêtement hydrophile pour la stabilisation d'une couche de liquide entre lentille artificielle et lentille naturelle.

13. Lentille intraoculaire selon l'une des revendications précédentes,
    **caractérisée en ce que**
    l'espace creux (3, 3') est réalisé sous forme de lentille à gaz, avec un gaz de remplissage ne diffusant pas à travers le matériau de lentille.

14. Lentille intraoculaire selon la revendication 13,
    **caractérisée en ce que**
    le gaz de remplissage est au moins l'un des gaz suivants :

    • dioxyde de carbone,
    • gaz rares, c'est-à-dire argon, néon, crypton, xénon, ainsi que :
    • hexafluorure de soufre,
    • octafluorpentane,

- perfluorobutane,
- perfluoropentane,
- octafluorocyclopentane,
- perfluorocyclopentane,
- perfluorométhylcyclopentane,
- perfluorocyclohexane,
- hydrofluoroéther,
- perfluorocétone, ou
- perfluorocyclohexane.

15. Lentille intraoculaire selon l'une des revendications précédentes,
    **caractérisée en ce que**
    pour assurer des compensations de pression/volume, le matériau de lentille est perméable, par pénétration par diffusion ou par extraction par diffusion pour des gaz atmosphériques, se présentant sous forme dissoute ou stockée sur des composants sanguins dans le corps humain, tels qu'en particulier l'oxygène ou l'azote.

16. Lentille intraoculaire selon l'une des revendications précédentes,
    **caractérisée par**
    des poids de ballast, en particulier des poids en forme d'anneau, dans les zones de bordure, pour assurer la compensation d'effets de flottabilité imputables au remplissage en gaz.

17. Lentille intraoculaire selon la revendication 16,
    **caractérisée en ce que**
    les poids de ballast sont reliés entre eux par une fixation mécaniquement stabilisante.

Fig. 1a

Fig. 1b

2b    3

2    1

2a

nicht akkommodiert

*Fig.2a*

2b    3    2c

2c    2

1

2a

akkommodiert

*Fig.2b*

6    3'    5

4

7

*Fig.3*

| Radien der Vorderfläche der biologischen Linse | Brechkraft der Vorderfläche ohne Gaslinse in Dioptrien | Brechkraft der Vorderfläche mit Gaslinse in Dioptrien | Akkommodative Leistung der biologischen Linse ohne Gaslinse in Dioptrien | Akkommodative Leistung der biologischen Linse mit Gaslinse in Dioptrien |
|---|---|---|---|---|
| 10,00 mm | 5 | 5 | 0 | 0 |
| 9,75 mm | 5,1282 | 5,99 | 0,182 | 0,99 |
| 9,50 mm | 5,263 | 7,06 | 0,263 | 2,03 |
| 9,25 mm | 5,405 | 8,129 | 0,405 | 3,129 |
| 9,00 mm | 5,55 | 9,29 | 0,55 | 4,29 |
| 8,75 mm | 5,71 | 10,514 | 0,71 | 5,514 |
| 8,50 mm | 5,88 | 11,81 | 0,88 | 6,81 |
| 8,00 mm | 6,25 | 14,65 | 1,25 | 9,65 |

Fig.4

**EP 2 124 821 B1**

**IN DER BESCHREIBUNG AUFGEFÜHRTE DOKUMENTE**

**In der Beschreibung aufgeführte Patentdokumente**

- FR 2666735